# FASCICULE DE BREVET EUROPEEN

(11) **EP 3 136 949 B1**
(45) Date de publication et mention de la délivrance du brevet: **31.01.2018**
(21) Numéro de dépôt: 15717926.8
(22) Date de dépôt: 28.04.2015
(51) Int. Cl.: A61B 5/00, B65D 75/30

(54) **DISPOSITIF POUR EVALUER LA PERCEPTION OROSENSORIELLE D'UN SUJET**
VORRICHTUNG ZUR AUSWERTUNG DER OROSENSORISCHEN WAHRNEHMUNG EINER PERSON
DEVICE FOR EVALUATING THE OROSENSORY PERCEPTION OF A SUBJECT

(30) Priorité: 28.04.2014 FR 1453796
(43) Date de publication de la demande: 08.03.2017
(73) Titulaire: Institut National de la Recherche Agronomique (INRA), 75007 Paris (FR)
(72) Inventeur: MARTIN, Christophe, 21000 Dijon (FR); ROBERT, Denis, 21121 Hauteville-les-Dijon (FR); FERON, Gilles, 21270 Pontailler sur Saone (FR)
(74) Mandataire: Jacobacci Coralis Harle
(86) Numéro de dépôt international: PCT/EP2015/059151
(87) Numéro de publication internationale: WO 2015/165880

(56) Documents cités:
- WO-A1-99/47431
- US-A1- 2010 228 098

## Description

### DOMAINE TECHNIQUE AUQUEL SE RAPPORTE L'INVENTION

La présente invention concerne un dispositif pour évaluer la perception orosensorielle d'un sujet à l'égard d'au moins un composé d'intérêt susceptible de constituer un stimulus orosensoriel.

### ARRIERE-PLAN TECHNOLOGIQUE

La perception sensorielle intéresse de nombreux domaines, notamment la recherche, l'analyse sensorielle ou l'éducation sensorielle.

Cette perception sensorielle résulte d'une intégration, par le cerveau d'un sujet, d'un ensemble de signaux (ou stimuli) transmis par ses sens : l'ouïe, le goût, le toucher, l'odorat et la vue.

Dans le cas d'un produit déposé dans la bouche d'un sujet, les stimuli sont libérés et perçus dans la sphère orosensorielle composée de la cavité buccale, du pharynx et de la cavité nasale.

La perception du sujet est alors du type « orosensorielle »: elle s'appuie sur les fonctions gustatives, olfactives et somesthésiques (en particulier la sensibilité chimique générale), liées respectivement à la perception des saveurs, des arômes et des composés trigéminaux.

En particulier, les saveurs (ou composés sapides) sont perçues par les récepteurs gustatifs situés sur la langue.

La dynamique de perception d'une saveur est principalement gouvernée par la libération temporelle de composés non volatiles qui se dissolvent dans la salive du sujet.

La perception des arômes (ou composés odorants) est quant à elle liée à la dynamique de libération de molécules volatiles odorantes.

Ces arômes doivent être libérés du produit afin d'atteindre les récepteurs olfactifs situés dans la cavité nasale.

Les organes de la sphère orosensorielle permettent également de percevoir des sensations dites « trigéminales » (informations véhiculées par le nerf trijumeau), telles que le piquant, l'irritant, l'astringent, etc., générées par des composés en contact avec des récepteurs situés dans les muqueuses.

L'évaluation de la perception gustative ou trigéminale chez un sujet s'effectue traditionnellement au moyen d'une gamme de solutions aqueuses contenant au moins un composé d'intérêt susceptible de constituer un stimulus gustatif ou trigéminal.

Chaque solution aqueuse est déposée directement dans la cavité buccale du sujet lors des tests d'évaluation.

Mais cette approche n'était pas entièrement satisfaisante, notamment sur le plan de la pertinence des résultats obtenus ou de l'ergonomie.

Il existe des dispositifs d'évaluation qui permettent de s'affranchir d'une telle solution aqueuse pour l'administration du composé d'intérêt, décrits par exemple dans les documents WO-99/47431 et US-2010/228098.

Les dispositifs d'évaluation correspondants comprennent classiquement un corps sur lequel sont rapportées des parties amovibles, par exemple des pastilles, qui sont imprégnées du composé d'intérêt.

Chaque pastille ou bande est déposée dans la cavité buccale du sujet pour évaluer sa perception gustative ou trigéminale à l'égard du composé d'intérêt en présence.

Mais, là encore, ces dispositifs ne sont pas entièrement ergonomiques : ils nécessitent un matériel et une procédure relativement complexes pour l'évaluation d'un sujet et pour éviter les erreurs de manipulation.

L'évaluation de la perception olfactive chez un sujet s'effectue traditionnellement au moyen de composés odorants sentis par voie ortho-nasale (directement par le nez, sans mise en bouche).

Un support solide (absorbant) ou liquide (solution) permet de diffuser ces composés odorants sous forme gazeuse (évaporation naturelle ou forcée).

Toutefois, ces dispositifs ne permettent pas de mettre les composés d'intérêt en bouche pour une perception rétro-nasale, plus proche de la perception des arômes des aliments.

En outre, de manière générale, les dispositifs d'évaluation actuels nécessitent une mise en oeuvre dans des conditions de laboratoires publics ou privés, ce qui oblige une logistique complexe et onéreuse.

Il existe par conséquent un besoin d'un dispositif d'évaluation qui serait d'une approche rapide, simple à utiliser, et surtout susceptible d'être applicable à grande échelle.

### OBJET DE L'INVENTION

La présente invention concerne un dispositif pour évaluer la perception orosensorielle d'un sujet offrant de nombreux avantages : une approche rapide, simple à utiliser, et susceptible d'être applicable à grande échelle.

A cet effet, le dispositif d'évaluation est du type comprenant un corps comportant au moins un composé d'intérêt susceptible de constituer un stimulus orosensoriel.

Le corps comprend au moins une partie amovible qui, d'une part, comporte ledit au moins un composé d'intérêt et, d'autre part, est adaptée à être séparée d'une partie résiduelle dudit corps pour être déposée dans la cavité buccale dudit sujet.

Selon l'invention, ledit corps est réalisé dans un matériau comestible ; ladite au moins une partie amovible est ménagée dans ledit corps, formant un ensemble monobloc, et ladite au moins une partie amovible est délimitée par une bordure périphérique dont au moins une portion est solidarisée avec une partie résiduelle dudit corps par une ligne de moindre résistance.

D'autres caractéristiques avantageuses de l'invention, pouvant être prises indépendamment ou en combinaison, sont indiquées ci-dessous :
- ladite ligne de moindre résistance consiste en une ligne de prédécoupe ;
- le corps est réalisé dans un matériau ayant un goût neutre ou quasiment neutre ;
- le corps consiste en une plaque, de préférence une feuille ;
- le matériau constitutif du corps, y compris les parties amovibles, est choisi parmi le papier azyme ;
- le dispositif d'évaluation comporte au moins une série d'évaluation formée d'au moins deux desdites parties amovibles : - l'une au moins desdites parties amovibles comportant le composé d'intérêt ou l'un au moins desdits composés d'intérêts, et - la ou les autres desdites parties amovibles (i) étant dépourvue(s) desdits composés d'intérêt (dite(s) partie(s) vierge(s)) ou (ii) comportant ledit ou lesdits composés d'intérêt, en combinaison avec au moins un composé complémentaire susceptible de modifier la perception orosensorielle ;
- le cas échéant, le dispositif d'évaluation comporte plusieurs séries d'évaluation successives dont les parties avec composé(s) d'intérêt respectives comportent des concentrations croissantes dudit ou desdits composés d'intérêt ;
- encore le cas échéant, les parties amovibles de chaque série d'évaluation sont ménagées sur une ligne, et lesdites séries sont réparties successivement sur le corps ;
- le corps comporte un marquage, de préférence visuelle ou en relief, qui est ménagé avantageusement de manière à délimiter et/ou à désigner la ou les parties amovibles dudit dispositif d'évaluation et/ou à faire figurer des informations ;
- le ou les composés d'intérêt sont choisis parmi les composés susceptibles de générer un stimulus des perceptions gustatives et/ou olfactives et/ou trigéminales du sujet ;
- le ou les composés d'intérêt sont de qualité alimentaire et sont solubles dans un solvant de qualité alimentaire choisi parmi l'eau et/ou un solvant organique et/ou leur mélange ;
- le dispositif d'évaluation est conditionné dans un emballage sous vide, de préférence un emballage aluminisé.

La présente invention concerne également un système pour évaluer la perception orosensorielle d'un sujet à l'encontre d'au moins un composé d'intérêt susceptible de constituer un stimulus orosensoriel, lequel ensemble d'évaluation comprend :
(i) un dispositif d'évaluation selon l'invention,
(ii) éventuellement un dispositif permettant une collecte écrite de données relatives à la perception orosensorielle dudit sujet au cours de la mise en oeuvre dudit dispositif d'évaluation, et
(iii) un programme d'ordinateur, enregistré sur un support lisible par un ordinateur, comprenant :
   - des moyens de programmation lisibles par un ordinateur pour la saisie desdites données relative à la perception orosensorielle dudit sujet et éventuellement pour la saisie de données personnelles dudit sujet,
   - des moyens de programmation lisibles par un ordinateur pour le traitement desdites données, aptes à déterminer un profil de perception orosensorielle, et
   - des moyens de programmation lisibles par un ordinateur pour la fourniture dudit profil de perception orosensorielle relatif audit sujet,
lorsque ledit programme d'ordinateur est exécuté sur un ordinateur.

La présente invention concerne également un procédé pour la fabrication d'un dispositif d'évaluation selon l'invention, comprenant :
- une étape de fourniture du corps apte à comporter le ou les composés d'intérêt, et
- une étape de dépose dudit ou desdits composés d'intérêt au sein d'au moins une partie dudit corps qui forme ou qui est destinée à former la ou les parties amovibles dudit corps,
lequel procédé comprend encore une étape de réalisation de ladite ou desdites parties amovibles dans le corps, avantageusement après l'étape de dépose.

Selon un mode de réalisation particulier, l'étape de réalisation de la ou des parties amovibles comprend une étape de réalisation de la ou des lignes de moindre résistance dans ledit corps pour délimiter ladite ou lesdites parties amovibles.

Selon encore un mode de réalisation particulier, l'étape de dépose consiste à déposer :
- au moins un composé d'intérêt choisi parmi les composés sapides ou les composés trigéminaux, sous forme d'une solution dont le solvant est constitué par de l'eau, ou
- au moins un composé d'intérêt choisi parmi les composés odorants, sous forme d'une solution dont le solvant est constitué par de l'eau ou une solution hydro-alcoolique.

Dans ce cas, la solution hydro-alcoolique contient un alcool, de préférence de l'éthanol

Avantageusement, l'étape de dépose s'effectue au moyen d'un dispositif d'impression du type jet d'encre, comprenant des réservoirs aptes à contenir des liquides et des moyens de projection des liquides contenus dans lesdits réservoirs, lesquels réservoirs contiennent respectivement :
- au moins un composé d'intérêt en solution dans un solvant,
- ledit solvant seul, et
- une encre liquide.

La présente invention concerne également un procédé pour évaluer la perception orosensorielle d'un sujet à l'encontre d'au moins un composé d'intérêt susceptible de constituer un stimulus orosensoriel.

Ce procédé d'évaluation comprend avantageusement les étapes suivantes :
- une étape de fourniture d'un dispositif d'évaluation selon l'invention,
- une étape de séparation d'une partie amovible par rapport à la partie résiduelle du corps,
- une étape de dépose de ladite partie amovible dans la cavité buccale dudit sujet, et
- une étape de collecte de données relatives à la perception orosensorielle dudit sujet.

Dans le cas d'un dispositif d'évaluation comportant au moins une série d'évaluation, ledit procédé comprend :
- une étape de dépose successive des parties amovibles d'une série d'évaluation dans la bouche du sujet,
- une étape d'identification par le sujet, parmi lesdites parties amovibles de la série d'évaluation, de la partie distinctive,
- une étape de rinçage de la bouche dudit sujet, et
- éventuellement une reproduction desdites étapes de dépose et d'identification pour une nouvelle série d'évaluation portée par ledit dispositif d'évaluation.

### DESCRIPTION DETAILLEE D'UN EXEMPLE DE REALISATION

L'invention est encore illustrée, sans être aucunement limitée, par la description suivante d'un mode de réalisation particulier, en relation avec les dessins annexés dans lesquels :
- la figure 1 est une vue de face d'un dispositif d'évaluation conforme à l'invention, consistant en une feuille munie de plusieurs parties amovibles qui sont agencées pour former plusieurs séries d'évaluation ;
- la figure 2 est un schéma bloc illustrant un procédé pour la fabrication du dispositif d'évaluation selon la figure 1 ;
- la figure 3 représente schématiquement les moyens pour déposer notamment le ou les composés d'intérêt sur le corps du dispositif d'évaluation selon la figure 1 ;
- la figure 4 représente schématiquement un système pour évaluer la perception orosensorielle d'un sujet, utilisant notamment le dispositif d'évaluation selon la figure 1.

Le dispositif selon l'invention est adapté à l'évaluation de la perception orosensorielle d'un sujet à l'égard d'au moins un composé d'intérêt susceptible de constituer un stimulus orosensoriel.

### Définition

Par « sujet », on entend (i) un sujet humain ou (ii) un sujet animal.

Par « animal », on entend en particulier un mammifère, choisi par exemple parmi les rongeurs (par exemple rat, souris, etc.).

Par « orosensoriel », on entend la sphère composée de la bouche, du pharynx et de la cavité nasale.

Par « perception orosensorielle » (ou « fonction orosensorielle ») d'un sujet, on entend la perception dudit sujet à l'égard d'un stimulus orosensoriel susceptible d'être détecté via sa sphère orosensorielle.

Dans le cadre de l'invention, cette perception orosensorielle englobe en particulier les informations collectées par certains sens du sujet, avantageusement :
- la perception gustative (ou le goût) et/ou
- la perception olfactive (ou l'odorat) et/ou
- la perception somesthésique, et en particulier la perception trigéminale.

Un « stimulus orosensoriel » consiste avantageusement en un composé qui, déposé dans la bouche (ou la cavité buccale) du sujet, est susceptible de faire l'objet d'une perception orosensorielle.

En particulier, pour rappel, la perception trigéminale (sensibilité chimique générale, et sensibilité tactile en particulier) relève de la perception somesthésique et permet de ressentir des sensations dites « trigéminales » (informations véhiculées par le nerf trijumeau), telles que le piquant, l'irritant, l'astringent, etc., générées par des composés en contact avec des récepteurs situés dans les muqueuses.

Un tel stimulus orosensoriel est avantageusement choisi parmi l'une des familles suivantes :
- les saveurs, dits encore « composés sapides » ou « stimuli sapides » (en particulier associées à la perception gustative), et/ou
- les arômes, dits encore « composés odorants » ou « stimuli olfactifs » ou « stimuli odorants » (en particulier associés à la perception olfactive), et/ou
- les composés trigéminaux (en particulier associés à la perception somesthésique et plus précisément la perception trigéminale).

Par « saveur », on entend en particulier un stimulus perçu par les récepteurs gustatifs situés sur la langue.

La dynamique de perception de la saveur est en particulier gouvernée par la libération temporelle de composés non volatiles qui se dissolvent dans la salive.

Par « saveur », on entend en particulier les saveurs de base : le sucre, le salé, l'acide, l'amer et l'umami. On entend aussi la sensation du gras (notamment les acides gras tels que l'acide oléique).

Par « arôme », on entend la perception liée à la dynamique de libération de molécules volatiles odorantes dans la sphère orosensorielle.

De tels stimuli olfactifs consistent généralement en des molécules volatiles qui doivent être libérés du produit afin d'atteindre les récepteurs olfactifs situés dans la cavité nasale.

Lorsque le composé d'intérêt est en bouche, cette perception s'effectue en particulier au travers de la voie « rétro-nasale ».

Par « composé trigéminal » (ou sensation trigéminale), on entend un composé générant une stimulation orosensorielle véhiculée par le nerf trijumeau et générant la perception d'une sensation dite « trigéminale » (piquant, brûlant, astringent, etc.).

Par « évaluer la perception orosensorielle d'un sujet », on entend en particulier une estimation de :
- sa sensibilité aux saveurs et/ou aux arômes et/ou aux sensations trigéminales, et/ou
- sa capacité à identifier et/ou à décrire des saveurs et/ou des arômes et/ou des sensations trigéminales, et/ou
- sa capacité à catégoriser des saveurs et/ou des arômes et/ou des sensations trigéminales, et/ou
- sa capacité à mémoriser des saveurs et/ou des arômes et/ou des sensations trigéminales.

Par « sensibilité aux saveurs et/ou aux arômes et/ou aux sensations trigéminales », on entend en particulier l'évaluation de :
- un seuil de détection, c'est-à-dire la capacité à différencier un stimulus faiblement intense par rapport à un échantillon dépourvu dudit stimulus ;
- un seuil de reconnaissance, c'est-à-dire la capacité à reconnaître et nommer un stimulus pour de faibles concentrations ;
- un seuil différentiel, c'est-à-dire la capacité à classer des échantillons selon l'intensité d'un stimulus orosensoriel.

Par « capacité à identifier des saveurs et/ou des arômes et/ou des sensations trigéminales », on entend en particulier la reconnaissance (nommer) ou la description verbale de ces stimuli.

Par « capacité à catégoriser des saveurs et/ou des arômes et/ou des sensations trigéminales », on entend en particulier le tri et le regroupement par similitude d'un certain nombre d'échantillons générant ces stimuli.

Par « capacité à mémoriser des saveurs et/ou des arômes et/ou des sensations trigéminales », on entend en particulier le stockage en mémoire de souvenirs plus ou moins récents liés à ces stimuli (stockage intentionnel ou non intentionnel), la conservation de ces souvenirs et leur rappel (implicite ou explicite). Cette évaluation de la perception orosensorielle permet avantageusement d'établir un « profil orosensoriel » dudit sujet.

Par « profil orosensoriel », on entend en particulier un ensemble de caractéristiques personnelles permettant de définir les capacités dudit sujet vis-à-vis de la perception des arômes et/ou des saveurs et/ou des sensations trigéminales, de comparer les sujets entre eux, et de les regrouper ou de les opposer en fonction de leurs ressemblances ou dissemblances.

### Sur le dispositif d'évaluation selon l'invention

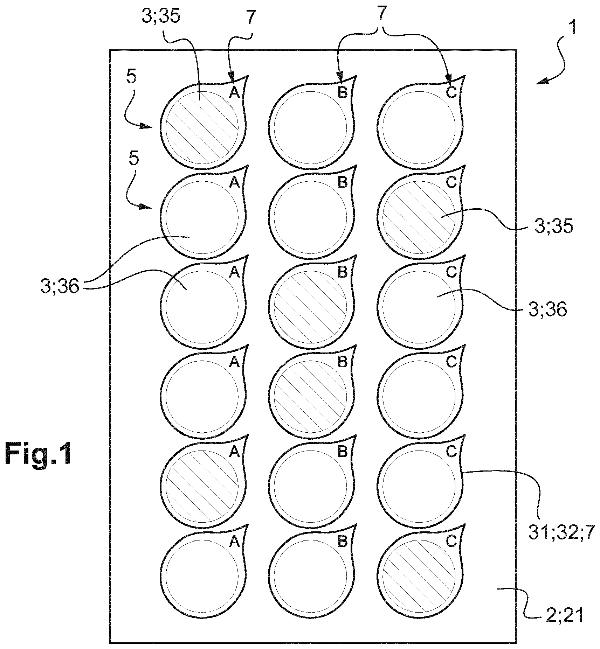

Le dispositif d'évaluation 1, représenté sur la figure 1, est avantageusement constitué d'un ensemble de produits/composés qui sont d'une qualité alimentaire.

Par « qualité alimentaire », on entend un produit comestible, apte à être pris en bouche par le sujet sans risque pour sa santé.

Par « comestible », on entend un produit consommable, qui peut être mangé (ou ingéré) par le sujet (avantageusement un sujet humain) sans risque pour sa santé.

En l'espèce, ce dispositif d'évaluation 1 comprend un corps 2 (ou matrice ou support) qui se présente ici sous la forme d'une plaque.

Selon l'invention, ce corps 2 comprend une partie support 21 (ou partie résiduelle) associée à une pluralité de parties amovibles 3 qui comportent le ou les composés d'intérêt susceptibles de constituer un stimulus orosensoriel.

### Le corps du dispositif d'évaluation

Le corps 2 se présente avantageusement sous la forme d'une plaque, et de préférence d'une feuille (plaque mince d'un matériau).

Cette plaque peut être rigide, semi-rigide ou souple.

Les surfaces opposées de ce corps 2 peuvent chacune être lisse ou en relief.

Le matériau constitutif du corps 2, y compris les parties amovibles 3 et la partie support 21 (ou partie résiduelle) précitées, sont comestibles.

Le matériau constitutif du corps 2 est par exemple choisi parmi le papier azyme.

Par « papier azyme » ou « papier hostie », on entend un mélange d'eau et de farine pétris ensemble, qui n'a pas gonflé sous l'effet du levain ou de la levure.

Par exemple, un tel papier azyme est constitué d'un mélange de fécule de pomme de terre, d'eau et d'huile végétale.

Le papier azyme a par exemple une épaisseur comprise entre 0,3 mm et 1,2 mm.

Plus généralement, le corps 2 est réalisé dans un matériau comestible, ayant avantageusement un goût neutre ou quasiment neutre.

Ce matériau présente en plus de préférence des propriétés mécaniques qui sont compatibles avec une dépose des composés d'intérêt, avantageusement au moyen d'une imprimante jet d'encre.

### Les parties amovibles du dispositif d'évaluation

Le corps 2 comprend au moins deux parties amovibles 3, dont l'une au moins comporte le ou les composés d'intérêt susceptibles de constituer un stimulus orosensoriel.

Les parties amovibles 3 sont adaptées à être séparées de la partie support 21 dudit corps 2 (constituant la partie résiduelle 21 du corps 2 après séparation) pour être déposées dans la cavité buccale du sujet.

Le corps 2 conserve une partie support ou résiduelle 21, après séparation de l'ensemble des parties amovibles 3.

Cette séparation est avantageusement aisée, manuellement ou au moyen d'un accessoire (par exemple un accessoire tranchant).

En particulier, les parties amovibles 3 sont ménagées directement dans le corps 2, pour former un ensemble monobloc avec la partie support 21.

Dans ce cas, les parties amovibles 3 consistent avantageusement chacune en une portion ou un morceau du corps 2, par exemple une portion ou un morceau de plaque et de préférence de feuille.

Encore dans ce cas, les parties amovibles 3 sont chacune délimitées par une bordure périphérique 31 dont au moins une portion (en tout ou partie) est solidarisée avec la partie support 21 par une ligne de moindre résistance 32 (ou ligne de faiblesse).

Cette ligne de moindre résistance 32 consiste avantageusement en une ligne de prédécoupe.

La ligne de prédécoupe 32 consiste par exemple en une petite languette non découpée de quelques millimètres de largeur (par exemple 3 à 4 mm).

Une simple torsion ou traction de la partie amovible 3, par rapport à la partie support 21, permet de la détacher (ou de la séparer) de cette partie support 21 du corps 2 (par une dégradation, et de préférence une déchirure, de la ligne de moindre résistance 32).

De manière générale, les parties amovibles 3 peuvent aussi être portées par la partie support 21, de sorte à être découpées au moyen d'un outil ou d'un instrument (ciseaux).

Les parties amovibles 3 ont une forme générale circulaire sur la figure 1, mais elles pourraient présenter toute autre forme générale (par exemple rectangulaire ou carrée) ou représenter le contour d'un objet (avantageusement la forme d'un aliment).

Ces parties amovibles 3 ont avantageusement une surface comprise entre 0,2 et 15 cm².

Les parties amovibles 3 comportent le ou les composés d'intérêt qui sont susceptibles de constituer un stimulus orosensoriel.

Le ou les composés d'intérêt sont avantageusement déposés en surface d'une partie amovible 3, voire sur au moins une partie de son épaisseur.

Chaque partie amovible 3 est avantageusement apte :
- à fixer le ou les composés d'intérêt, par exemple par adsorption, et
- à libérer le ou les composés d'intérêt lorsqu'elle est plongée dans un liquide, avantageusement dans la salive produite dans la cavité buccale d'un sujet.

### Les composés d'intérêt

Les composés d'intérêt sont avantageusement choisis parmi les composés naturels et/ou les composés synthétiques et/ou les composés semi-synthétiques, susceptibles de constituer un stimulus orosensoriel.

En particulier, de tels composés d'intérêt sont de préférence choisis parmi les composés sapides et/ou odorants et/ou trigéminaux.

Par exemple, pour évaluer la sensibilité de sujets à la saveur amère, les parties amovibles 3 du dispositif d'évaluation 1 peuvent présenter plusieurs variantes qui se différencient par la nature du composé sapide utilisé : quinine, caféine, naringine, etc.

Les parties amovibles 3 du dispositif d'évaluation 1 pour la sensibilité à la saveur sucrée peuvent comporter l'un des composés sapides suivants : saccharose, fructose, glucose, aspartame, acésulfame K, etc.

Pour les composés odorants, on peut par exemple utiliser des composés purs (menthol, géraniol, etc.), des composés en mélange (mélanges binaires, ternaires, etc.), des compositions aromatiques complexes, des extraits tels que des huiles essentielles.

Pour les composés trigéminaux, on utilise par exemple des tanins (astringence), de la pipérine (piquant / brûlant).

De manière générale, là encore, les composés d'intérêt sont de qualité alimentaire.

De préférence, en vue de faciliter leur application, ces composés d'intérêt sont solubles dans l'eau et/ou dans un solvant organique alimentaire et/ou dans un mélange de ces solvants.

En fonction de l'évaluation orosensorielle souhaitée, la ou les parties amovibles 3 du dispositif d'évaluation 1 peuvent comporter :
(i) un unique composé d'intérêt, ou
(ii) une combinaison d'au moins deux composés d'intérêt.

Par « combinaison », on entend en particulier une combinaison :
- d'au moins deux composés d'intérêt d'un même type (sapides ou odorants ou trigéminaux), ou
- d'au moins deux composés d'intérêt de types différents (par exemple un composé sapide et un composé odorant).

Les différentes parties amovibles 3 peuvent également comporter :
- le même ou les mêmes composés d'intérêt, dans des concentrations différentes les unes par rapport aux autres, par exemple pour tester la sensibilité de sujets à un stimulus orosensoriel, et/ou
- des composés d'intérêt qui différent les unes par rapport aux autres, par exemple pour tester la capacité de sujets à identifier/reconnaitre des stimuli orosensoriels.

Selon une variante, certaines parties amovibles 3 peuvent également comporter au moins un composé complémentaire qui est susceptible de modifier la perception orosensorielle du sujet à l'égard dudit ou desdits composés d'intérêt en présence.

Le composé complémentaire est avantageusement choisi parmi les composés susceptibles de modifier la perception gustative et/ou la perception olfactive et/ou la perception trigéminale.

### Séries d'évaluation

L'agencement des parties amovibles 3 est adapté à façon, notamment en fonction de l'évaluation de perception orosensorielle recherchée.

Comme illustré sur la figure 1, uniquement à titre d'exemple non limitatif, les parties amovibles 3 sont avantageusement agencées sur le corps 2 pour former une pluralité de séries d'évaluation 5.

Un tel mode de réalisation est intéressant en particulier pour tester la sensibilité de sujets à un stimulus orosensoriel.

Chaque série d'évaluation 5 comprend au moins deux parties amovibles 3 (en l'occurrence trois).

Les séries d'évaluations 5 sont ici agencées chacune sur une ligne et sont ménagées parallèlement les unes aux autres, réparties successivement sur la hauteur du corps 2.

Avantageusement, l'une des parties amovibles 35, « distinctive » ou « intrus » (hachurée sur la figure 1), comporte des composés qui diffèrent par rapport aux autres parties amovibles 36 « référentielles (non hachurées sur la figure 1) de la série d'évaluation 5.

Cette approche vise à évaluer la capacité du sujet à distinguer la partie amovible distinctive 35 par rapport aux parties amovibles référentielles 36 de la série d'évaluation 5.

Les parties amovibles distinctives 35 sont distinguées sur la figure 1 uniquement dans un souci d'explication. Mais en pratique, de préférence, ces parties amovibles distinctives 35 ne sont pas identifiables (par le sujet notamment) parmi les autres parties amovibles 3.

De manière alternative, les parties amovibles 3 peuvent comporter des couleurs différentes de manière à évaluer les interactions entre la perception visuelle et la perception orosensorielle.

Selon un premier mode de réalisation, chaque série d'évaluation 5 comprend avantageusement :
- une partie amovible 35 distinctive « stimulus », comportant un ou plusieurs desdits composés d'intérêt, et
- au moins une partie amovible 36 référentielle « vierge », ici au nombre de deux, dépourvue desdits composés d'intérêt.

Au sein de chacune de ces séries d'évaluation 5, la partie amovible 35 distinctive est répartie aléatoirement et d'une manière non-identifiable par le sujet.

De telles séries d'évaluation 5 sont notamment intéressantes pour évaluer la sensibilité (seuil de détection) à un composé d'intérêt.

Pour cela, les parties amovibles 35 distinctives des séries d'évaluation 5 successives comportent le ou les mêmes composés d'intérêt, mais dans une gamme croissante de concentration.

Des prétests permettent de centrer cette gamme sur une concentration générant un stimulus perçu par environ 50 % de la population. La concentration la plus forte devra être perçue par plus de 90 % de la population. La concentration la plus faible devra être perçue par moins de 10 % de la population.

Pour un test de reconnaissance d'odeur, il est avantageux de déterminer des concentrations à la fois iso-intenses et perceptibles par l'ensemble de la population.

Selon un second mode de réalisation, les parties amovibles 3 d'une série d'évaluation 5 comprennent chacune le même ou les mêmes composés d'intérêt.

La partie amovible 35 distinctive comporte, en plus, au moins un composé complémentaire susceptible de modifier la perception orosensorielle à l'égard du ou des composés d'intérêt en présence.

Par « modifier la perception orosensorielle », on entend en particulier une augmentation ou une diminution (effet rehausser ou masquant, respectivement) de la perception à l'égard du ou des composés d'intérêt en présence.

De tels composés complémentaires sont par exemple utilisés pour masquer l'amertume (édulcorants, arômes) ou pour rehausser une saveur (par exemple arôme bacon pour rehausser une note salée).

D'autres modes de réalisation sont envisageables en fonction des capacités évaluées : sensibilité, identification / reconnaissance, catégorisation, mémorisation des stimuli.

### Marquage du dispositif d'évaluation

Le corps 2 comporte avantageusement un ensemble de marquages 7 qui sont ménagés de manière faire apparaître différentes informations sur le dispositif d'évaluation 1, par exemple :
- des traits longeant la bordure périphérique 31 de chacune des parties amovibles 3,
- la désignation des parties amovibles 3 et/ou des séries d'évaluation 5.

De tels marquages 7 peuvent être visuels et/ou tactiles (par exemple du type braille).

Par exemple, le marquage visuel 7 est une encre alimentaire.

### Conditionnement du dispositif d'évaluation

Le dispositif d'évaluation 1 est avantageusement conservé à l'abri de la lumière et de l'humidité.

A cet égard, il est avantageusement conservé dans un emballage sous vide (non représenté).

Pour des dispositifs d'évaluation de la perception olfactive, un emballage aluminisé permet de limiter les pertes en composés odorants qui sont volatiles.

### Sur le procédé et l'installation pour la fabrication du dispositif d'évaluation selon l'invention

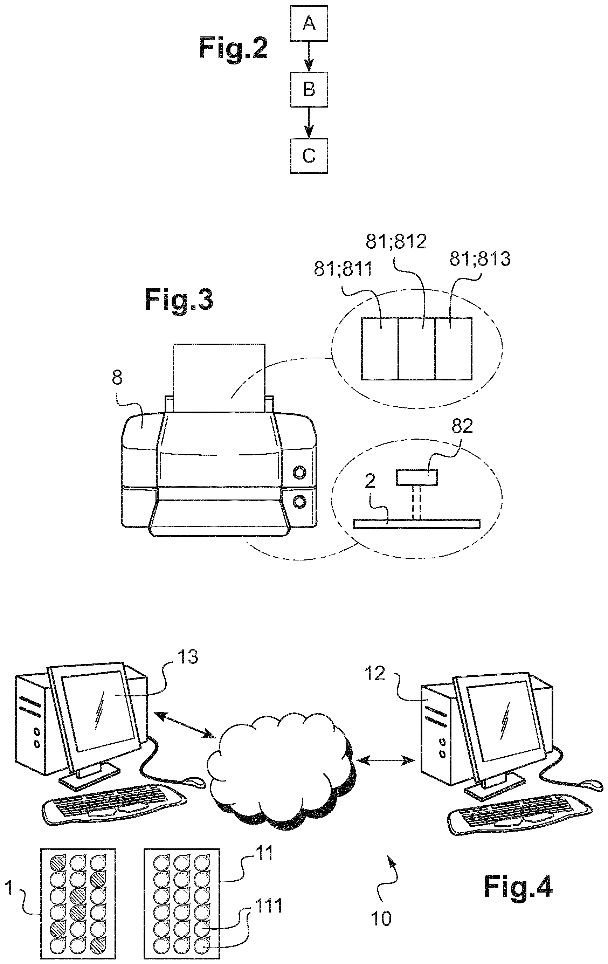

Le procédé et l'installation pour la fabrication d'un dispositif d'évaluation 1 selon l'invention sont décrits ci-dessous en relation avec les figures 2 et 3.

En pratique, le procédé selon l'invention commence par une étape de fourniture du corps 2 (étape A), en l'occurrence une feuille de qualité alimentaire apte à recevoir le ou les composés d'intérêt.

Le corps 2 est ici dépourvu des parties amovibles 3 et des composés d'intérêt.

L'étape de fourniture A est suivie par une étape B au cours de laquelle sont déposés sur l'une au moins de ses faces (une face ou les deux faces), en une seule passe ou plusieurs passes successives :
- les composés d'intérêt au sein de zones du corps 2 qui sont destinées à correspondre aux parties amovibles 3 du dispositif d'évaluation 1, et
- le marquage visuel 7.

Cette étape de dépose B s'effectue avantageusement au moyen d'un dispositif d'impression de type à jet d'encre 8 (dit encore « imprimante jet d'encre »), qui est couramment utilisé par exemple en pâtisserie.

Un tel dispositif d'impression 8, représenté schématiquement sur la figure 3, comprend avantageusement plusieurs réservoirs 81 et des moyens 82 pour la projection des liquides contenus dans lesdits réservoirs 81 (par exemple une tête d'imprimante jet d'encre).

Dans le cas de séries d'évaluation 5 comportant des parties amovibles 3 stimulus/vierges, les réservoirs 81 sont avantageusement chacun aptes à contenir des liquides à projeter, à savoir respectivement :
- un premier réservoir 811 destiné à contenir au moins un composé d'intérêt en solution dans un solvant, pour l'application dans les parties amovibles 35 stimulus précitées,
- un deuxième réservoir 812 destiné à contenir le solvant seul, pour l'application en particulier dans les parties amovibles 36 vierges, et
- un troisième réservoir 813 destiné à contenir une encre liquide de qualité alimentaire, pour l'application du marquage visuel 7.

Le solvant utilisé est avantageusement :
- soit de l'eau,
- soit un solvant organique alimentaire (par exemple un alcool et de préférence de l'éthanol),
- soit un mélange de ces deux solvants, dans des proportions variables.

Le solvant est en particulier choisi en fonction de la solubilité du ou des composés d'intérêt à déposer.

Par exemple, le solvant pour les composés sapides ou trigéminaux est avantageusement l'eau, de préférence l'eau déminéralisée.

Le solvant utilisé pour les composés odorants est de préférence soit l'eau déminéralisée, soit une solution hydro-alcoolique dont l'alcool est avantageusement de l'éthanol.

La concentration en solvant organique alimentaire dans un mélange est adaptée en fonction des affinités des composés d'intérêt avec l'eau.

Avantageusement, les solutions comportent une proportion minimum d'eau avoisinant les 20%, pour un fonctionnement optimal de dispositif d'impression 8.

La quantité en composé(s) d'intérêt déposée sur les parties amovibles 3, et par conséquent sa concentration surfacique, dépend d'une combinaison de deux paramètres :
- la concentration volumique en composé(s) d'intérêt de la solution présente dans le réservoir 811 correspondant, et
- le réglage de la quantité de solution déposée.

Suite à l'étape de dépose B, le corps 2 est soumis une étape de formation des parties amovibles 3 (une étape C).

Cette étape C de formation des parties amovibles 3 est par exemple effectuée à l'aide d'un emporte-pièce ou au moyen d'un dispositif du type automate programmable muni de moyens de découpe (traceur de découpe ou laser, par exemple), qui vient appliquer une découpe partielle (ou prédécoupe) dans l'épaisseur du corps 2.

Cette étape C de formation des parties amovibles 3 permet de ménager les lignes de moindre résistance 32 délimitant chacune des parties amovibles 3.

Pour finir, le cas échéant, le dispositif d'évaluation 1 selon l'invention est conditionné individuellement ou en lot.

De manière alternative, l'étape C de formation des parties amovibles 3 pourrait être mise en oeuvre avant l'étape de dépose B.

### Mise en oeuvre du dispositif d'évaluation selon l'invention

Le dispositif d'évaluation 1 selon l'invention est ainsi utile pour l'évaluation de la perception orosensorielle d'un sujet à l'encontre d'un composé d'intérêt susceptible de constituer un stimulus orosensoriel.

Le dispositif d'évaluation 1 décrit ci-dessus en relation avec la figure 1 est intéressant dans le cadre d'un test de sensibilité.

Dans ce cadre, la succession d'étapes suivantes est avantageusement mise en oeuvre :
- une étape de dépose successive des parties amovibles 3 d'une série d'évaluation 5 dans la bouche du sujet,
- une étape d'identification (ou au moins de tentative d'identification) par le sujet, parmi lesdites parties amovibles 3 de la série d'évaluations 5, de la partie amovible 35 distinctive par rapport aux autres parties amovibles 36 de ladite série 5, et
- une étape de rinçage de la bouche dudit sujet, par exemple avec de l'eau.

Pour rappel, la partie amovible 35 distinctive, à identifier par le sujet, peut correspondre par exemple :
- à une partie stimulus parmi des parties vierges, ou
- à une partie amovible portant une combinaison composé d'intérêt / composé complémentaire, parmi des parties amovibles comportant uniquement le composé d'intérêt.

Ces différentes étapes sont reproduites pour les séries d'évaluation 5 successives portées par le dispositif d'évaluation 1.

Les résultats collectés peuvent être traités sur une méthode préétablie ou une méthode à façon, de sorte à obtenir un profil de perception orosensorielle dudit sujet.

De manière générale, le dispositif d'évaluation 1 selon l'invention, et sa mise en oeuvre, peuvent être adaptés à façon, tenant compte notamment de l'évaluation sensorielle recherchée chez les sujets.

Par exemple, le dispositif d'évaluation 1 peut être adapté à façon pour évaluer - la sensibilité aux saveurs et/ou aux arômes et/ou aux sensations trigéminales, et/ou - la capacité à identifier et/ou à décrire des saveurs et/ou des arômes et/ou des sensations trigéminales, et/ou - la capacité à catégoriser des saveurs et/ou des arômes et/ou des sensations trigéminales, et/ou - la capacité à mémoriser des saveurs et/ou des arômes et/ou des sensations trigéminales.

### Sur le système pour évaluer la perception orosensorielle d'un sujet

En pratique, le dispositif d'évaluation 1 fait avantageusement partie d'un système qui se présente sous la forme d'un ensemble prêt-à-utiliser (« kit »).

Le système d'évaluation 10 selon l'invention (figure 4) comprend avantageusement :
(i) un dispositif d'évaluation 1 tel que décrit ci-dessus,
(ii) éventuellement un dispositif 11 permettant une collecte écrite de données relatives à la perception orosensorielle du sujet au cours de la mise en oeuvre du dispositif d'évaluation 1, et
(iii) un programme d'ordinateur (non représenté) pour le traitement et la fourniture d'un profil de perception orosensorielle, tenant compte des données issues de la mise en oeuvre du dispositif d'évaluation 1.

Le dispositif de collecte 11 consiste avantageusement en une feuille « réponse », reprenant exactement l'esthétique du dispositif d'évaluation 1.

En particulier, des zones 111 sont dessinées sur cette feuille de réponse 11 de telle sorte à reproduire l'esthétisme et le marquage 7 des parties amovibles 3 du dispositif d'évaluation 1.

Une telle feuille de réponse 11 permet au sujet de noter ses réponses, avant de les reporter dans le programme d'ordinateur.

Une telle feuille de réponse 11 est en particulier utile lorsque le sujet évalué est dans l'incapacité de saisir ses réponses directement dans ledit programme d'ordinateur.

Le programme d'ordinateur du système selon l'invention consiste quant à lui avantageusement en une « application Web », c'est-à-dire un programme d'ordinateur présent sur un ordinateur distant 12 et manipulable via Internet grâce à un logiciel de navigation (ou navigateur Web) présent sur un ordinateur local 13.

L'ordinateur local 13 peut consister par exemple en un micro-ordinateur classique ou en un ordinateur portable ou en un dispositif portatif personnel (tablette numérique, téléphone portable, etc.).

Ce programme d'ordinateur, enregistré sur un support lisible par un ordinateur (par exemple un disque dur), comprend avantageusement :
- des moyens de programme lisibles par un ordinateur, pour la saisie de données relatives à la perception orosensorielle du sujet et éventuellement pour la saisie de données personnelles dudit sujet (sexe, âge),
- des moyens de programme lisibles par un ordinateur, pour le traitement desdites données, aptes à déterminer un profil de perception orosensorielle, et
- des moyens de programme lisibles par un ordinateur, pour la fourniture dudit profil de perception orosensorielle relatif audit sujet,
lorsque ledit programme d'ordinateur est exécuté sur un ordinateur.

Par « moyens de programme », on entend en particulier des portions ou des instructions de code de programme d'ordinateur.

Par « profil de perception orosensorielle », on entend en particulier un « score de performances » ou un diagnostic établi selon une norme.

Par « score de performance », on entend un score basé sur les réponses données, permettant de chiffrer les capacités sensorielles des sujets.

Par « diagnostic établi selon une norme », on entend la comparaison des réponses données par rapport à des données portant sur un nombre suffisamment important de sujets pour constituer une référence (par exemple, un score moyen calculé à partir des résultats de 1000 sujets représentatifs de la population peut servir de référence pour déterminer si le score obtenu par un sujet permet de le classer parmi les sujets plutôt sensibles / peu sensibles).

### Applications envisageables

Le dispositif selon l'invention peut être utile pour la recherche, l'analyse sensorielle, l'éducation sensorielle et le jeu.

Pour la recherche :
- phénotypage sensoriel des populations et mise en relation des données avec d'autres types de données recueillis (par exemple dans le cadre des grandes cohortes liées à des projets de recherche centrée sur la nutrition, la santé et le comportement alimentaire : données physiologiques, pathologies diverses, comportement alimentaire, caractéristiques socio-démographiques, culture, tabagisme, etc.) ;
- établissement de normes précisant les performances sensorielles attendues en fonction de différents critères (âge, sexe, etc.) ;
- détection des troubles gustatifs et olfactifs, étude de population suivant un traitement médical, aide à la détection précoce de certaines maladies (maladies neurodégénératives en particulier) ;
- rééducation neurologique par les odeurs : mise en place d'ateliers olfactifs permettant de faire travailler la mémoire par l'intermédiaire des odeurs ;
- étude des interactions entre modalités sensorielles (disque présentant des stimuli multiples).

Pour l'analyse sensorielle :
- sélection des panélistes présentant une performance sensorielle satisfaisante lors de la constitution d'un jury d'analyse sensorielle (test de sensibilité, test de reconnaissance) ;
- utilisation de dispositifs d'évaluation illustrant des standards d'intensité (arôme ou saveur) ;
- test consommateur en condition d'achat (lieu de vente);
- évaluation des propriétés sapides ou aromatiques de substitut sensoriel (édulcorant, substitut du sel).

Pour l'éducation sensorielle et le jeu :
- dispositif d'évaluation ou jeu utilisable dans les écoles, sans préparation pour les professeurs des écoles (éveil aux « goût ») ;
- dispositif d'évaluation utilisable dans les grandes manifestations (Salon de l'Agriculture, Foires Gastronomiques, etc.) ;
- jeux éducatifs sur les saveurs et les arômes.

Le dispositif selon l'invention permet également de caractériser les attitudes (préférences, aversions) du sujet vis-à-vis de la perception orosensorielle, notamment de saveurs, d'arômes et de sensations trigéminales.

Le dispositif d'évaluation selon l'invention peut également être intéressant pour évaluer les préférences (notamment pour la saveur sucrée) dans le cadre de l'expérimentation animale.

De tels dispositifs peuvent guider les expérimentateurs dans la procédure à suivre pour tester l'animal (ordre dans lequel il faut distribuer les parties amovibles).

La faible teneur énergétique des parties amovibles et leur goût neutre sont des avantages par rapport à d'autres aliments utilisés pour les tests avec les animaux (pâtées par exemple).

Le dispositif d'évaluation selon l'invention offre la possibilité de pouvoir proposer un outil fiable et flexible, pouvant être utilisé en tout lieu, ce qui constitue un intérêt pour les acteurs socio-économiques du domaine.

Le dispositif selon l'invention est particulièrement adapté pour un envoi par courrier, pouvant être utilisé à domicile, en l'absence d'un expérimentateur.

Le dispositif selon l'invention présente encore les avantages suivants :
- prêt à l'emploi, ne nécessitant pas d'encadrement par une personne expérimentée,
- rapidité d'exécution,
- utilisation possible à grande échelle,
- envoi possible par courrier,
- obtention de résultats et de diagnostics par Internet pour l'utilisateur,
- constitution progressive d'une base de données pour l'expérimentateur,
- fabrication rapide.

## Revendications

1. Dispositif pour évaluer la perception orosensorielle d'un sujet, lequel dispositif d'évaluation (1) comprend un corps (2) comportant au moins un composé d'intérêt susceptible de constituer un stimulus orosensoriel,
lequel corps (2) comprend au moins une partie amovible (3) qui, d'une part, comporte ledit au moins un composé d'intérêt et, d'autre part, est adaptée à être séparée d'une partie résiduelle (21) dudit corps (2) pour être déposée dans la cavité buccale dudit sujet,
laquelle au moins une partie amovible (3) est ménagée dans ledit corps (2), formant un ensemble monobloc,
laquelle au moins une partie amovible (3) est délimitée par une bordure périphérique (31) dont au moins une portion est solidarisée avec une partie résiduelle (21) dudit corps (2),
**caractérisé en ce que** ledit corps (2) est réalisé dans un matériau comestible, et
**en ce que** au moins une portion de ladite bordure périphérique (31) de ladite au moins une partie amovible (3) est solidarisée avec ladite partie résiduelle (21) dudit corps (2) par une ligne de moindre résistance (32).

2. Dispositif d'évaluation selon la revendication 1, **caractérisé en ce que** ladite ligne de moindre résistance (32) consiste en une ligne de prédécoupe.

3. Dispositif d'évaluation selon l'une quelconque des revendications 1 ou 2, **caractérisé en ce que** le corps (2) est réalisé dans un matériau ayant un goût neutre ou quasiment neutre.

4. Dispositif d'évaluation selon l'une quelconque des revendications 1 à 3, **caractérisé en ce que** le corps (2) consiste en une plaque, de préférence une feuille.

5. Dispositif d'évaluation selon l'une quelconque des revendications 1 à 4, **caractérisé en ce que** le matériau constitutif du corps (2), y compris les parties amovibles (3), est le papier azyme.

6. Dispositif d'évaluation selon l'une quelconque des revendications 1 à 5, **caractérisé en ce qu'**il comporte au moins une série d'évaluation (5) formée d'au moins deux desdites parties amovibles (3) :
- l'une au moins desdites parties amovibles (3) comportant le composé d'intérêt ou l'un au moins desdits composés d'intérêts, et
- la ou les autres desdites parties amovibles (3) (i) étant dépourvue(s) desdits composés d'intérêt ou (ii) comportant ledit ou lesdits composés d'intérêt, en combinaison avec au moins un composé complémentaire susceptible de modifier la perception orosensorielle.

7. Dispositif d'évaluation selon l'une quelconque des revendications 1 à 6, **caractérisé en ce que** le corps (2) comporte un marquage (7) qui est ménagé de manière à délimiter et/ou à désigner ladite au moins une partie amovible (3) dudit dispositif d'évaluation (1).

8. Dispositif d'évaluation selon l'une quelconque des revendications 1 à 7, **caractérisé en ce que** ledit au moins un composé d'intérêt est choisi parmi les composés susceptibles de générer un stimulus des perceptions gustatives et/ou olfactives et/ou trigéminales du sujet.

9. Système pour évaluer la perception orosensorielle d'un sujet à l'encontre d'au moins un composé d'intérêt susceptible de constituer un stimulus orosensoriel, lequel ensemble d'évaluation (10) comprend :
(i) un dispositif d'évaluation (1) selon l'une quelconque des revendications 1 à 8,
(ii) éventuellement un dispositif (11) permettant une collecte écrite de données relatives à la perception orosensorielle dudit sujet au cours de la mise en oeuvre dudit dispositif d'évaluation (1), et
(iii) un programme d'ordinateur, enregistré sur un support lisible par un ordinateur, comprenant :
- des moyens de programmation lisibles par un ordinateur pour la saisie desdites données relative à la perception orosensorielle dudit sujet et éventuellement pour la saisie de données personnelles dudit sujet,
- des moyens de programmation lisibles par un ordinateur pour le traitement desdites données, aptes à déterminer un profil de perception orosensorielle, et
- des moyens de programmation lisibles par un ordinateur pour la fourniture dudit profil de perception orosensorielle relatif audit sujet,
lorsque ledit programme d'ordinateur est exécuté sur un ordinateur.

10. Procédé pour la fabrication d'un dispositif d'évaluation (1) selon l'une quelconque des revendications 1 à 9, **caractérisé en ce qu'**il comprend :
- une étape (A) de fourniture du corps (2) apte à comporter ledit au moins un composés d'intérêt, et
- une étape (B) de dépose dudit au moins un composé d'intérêt au sein d'au moins une partie dudit corps (2) qui forme ou qui est destinée à former ladite au moins une partie amovible (3) dudit corps (2),
lequel procédé comprend encore une étape (C) de réalisation de ladite au moins une partie amovibles (3) dans le corps (2).

11. Procédé de fabrication selon la revendication 10, **caractérisé en ce que** l'étape (C) de réalisation de ladite au moins une partie amovible (3) comprend une étape de réalisation de ladite au moins une ligne de moindre résistance (32) dans ledit corps (2) pour délimiter ladite au moins une partie amovible (3).

12. Procédé de fabrication selon l'une quelconque des revendications 10 ou 11, **caractérisé en ce que** l'étape de dépose (B) consiste à déposer :
- au moins un composé d'intérêt choisi parmi les composés sapides ou les composés trigéminaux, sous forme d'une solution dont le solvant est constitué par de l'eau, ou
- au moins un composé d'intérêt choisi parmi les composés odorants, sous forme d'une solution dont le solvant est constitué par de l'eau ou une solution hydro-alcoolique.

13. Procédé de fabrication selon l'une quelconque des revendications 10 à 12, **caractérisé en ce que** l'étape de dépose (B) s'effectue au moyen d'un dispositif d'impression (8) du type jet d'encre, comprenant des réservoirs (81) aptes à contenir des liquides et des moyens (82) de projection des liquides contenus dans lesdits réservoirs (81),
lesquels réservoirs (81) contiennent respectivement :
- au moins un composé d'intérêt en solution dans un solvant,
- ledit solvant seul, et
- une encre liquide.

## Patentansprüche

1. Vorrichtung zur Auswertung der orosensorischen Wahrnehmung einer Person, wobei die Auswertungsvorrichtung (1) einen Körper (2) aufweist, der wenigstens eine Verbindung von Interesse aufweist, die einen orosensoriellen Stimulus bilden kann,
wobei der Körper (2) wenigstens einen abnehmbaren Teil (3) aufweist, der einerseits die wenigstens eine Verbindung von Interesse enthält und andererseits dazu ausgelegt ist, von einem restlichen Teil (21) des Körpers (2) getrennt zu werden, um in der Mundhöhle der Person abgelegt zu werden,
wobei der wenigstens eine abnehmbare Teil (3) im Körper (2) eingerichtet ist und mit diesem eine einstückige Einheit bildet, wobei der wenigstens eine abnehmbare Teil (3) durch einem umlaufenden Rand (31) begrenzt ist, von dem wenigstens ein Teil mit einem restlichen Teil (21) des Körpers (2) fest verbunden ist,
**dadurch gekennzeichnet, daß** der Körper (2) aus einem eßbaren Material gefertigt ist und daß wenigstens ein Teil des umlaufenden Rands (31) des wenigstens einen abnehmbaren Teils (3) mit dem restlichen Teil (21) des Körpers (2) durch eine Linie mit verringerter Festigkeit (32) verbunden ist.

2. Auswertungsvorrichtung gemäß Anspruch 1, **dadurch gekennzeichnet, daß** die Linie mit verringerter Festigkeit (32) aus einer vorgeschnittenen Linie besteht.

3. Auswertungsvorrichtung gemäß einem der Ansprüche 1 oder 2, **dadurch gekennzeichnet, daß** der Körper (2) aus einem Material mit neutralem oder fast neutralem Geschmack gefertigt ist.

4. Auswertungsvorrichtung gemäß einem der Ansprüche 1 bis 3, **dadurch gekennzeichnet, daß** der Körper (2) aus einer Platte, vorzugsweise aus einem Blatt besteht.

5. Auswertungsvorrichtung gemäß einem der Ansprüche 1 bis 4, **dadurch gekennzeichnet, daß** das den Körper (2) bildende Material inklusive der abnehmbaren Teile (3) Eßpapier ist.

6. Auswertungsvorrichtung gemäß einem der Ansprüche 1 bis 5, **dadurch gekennzeichnet, daß** sie wenigstens eine aus wenigstens zwei der abnehmbaren Teile gebildete Auswertungsreihe (5) aufweist:
- wobei wenigstens eins der abnehmbaren Teile (3) die Verbindung von Interesse oder wenigstens eine der Verbindungen von Interesse aufweist und
- wobei der andere oder die anderen abnehmbare(n) Teil(e) (3) (i) entweder die Verbindung von Interesse nicht aufweist (aufweisen) oder (ii) die Verbindung oder Verbindungen von Interesse zusammen mit wenigstens einer komplementären, die orosensorielle Wahrnehmung eventuell verändernde Verbindung aufweist (aufweisen).

7. Auswertungsvorrichtung gemäß einem der Ansprüche 1 bis 6, **dadurch gekennzeichnet, daß** der Körper (2) eine Markierung (7) aufweist, die so angelegt ist, daß sie den wenigstens einen abnehmbaren Teil (3) der Auswertungsvorrichtung (1) begrenzt und/oder bezeichnet.

8. Auswertungsvorrichtung gemäß einem der Ansprüche 1 bis 7, **dadurch gekennzeichnet, daß** die wenigstens eine Verbindung von Interesse unter den Verbindungen ausgewählt ist, die einen Stimulus der geschmacksmäßigen und/oder geruchsmäßigen und/oder trigeminalen Wahrnehmungen der Person erzeugen kann.

9. System zur Auswertung der orosensorischen Wahrnehmung einer Person hinsichtlich wenigstens einer Verbindung ausgewählt ist, die einen Stimulus der geschmacksmäßigen und/oder geruchsmäßigen und/oder trigeminalen Wahrnehmungen der Person erzeugen kann, wobei das Auswertungssystem (10) folgendes aufweist:
(i) eine Auswertungsvorrichtung gemäß einem der Ansprüche 1 bis 8,
(ii) eventuell eine Vorrichtung (11), die eine geschriebene Sammlung von Daten bezüglich der sensoriellen Wahrnehmung der Person während der Verwendung der Auswertungsvorrichtung (1) ermöglicht, und
(iii) ein auf einem durch einen Computer lesbaren Speicher gespeichertes Computerprogramm, das
- durch einen Computer lesbare Programmiermittel für das Erfassen der Daten bezüglich der orosensoriellen Wahrnehmung der Person und eventuell für das Erfassen persönlicher Daten der Person,
- durch einen Computer lesbare Programmiermittel für das Verarbeiten der Daten, die geeignet sind, ein Profil der orosensoriellen Wahrnehmung zu bestimmen, und
- durch einen Computer lesbare Programmiermittel für das Bereithalten des Profils der orosensoriellen Wahrnehmung bezüglich der Person
aufweist.

10. Verfahren zur Herstellung einer Auswertungsvorrichtung (1) gemäß einem der Ansprüche 1 bis 9, **dadurch gekennzeichnet, daß** es
- einen Schritt (A) des Bereitstellens des Körpers (2), der geeignet ist, die wenigstens eine Verbindung von Interesse aufzuweisen,
- einen Schritt (B) des Ablegens der wenigstens einen Verbindung von Interesse auf wenigstens einem Teil des Körpers (2), der den wenigstens einen abnehmbaren Teil (3) des Körpers (2) bildet oder bilden soll,
aufweist,
wobei das Verfahren noch einen Schritt (C) des Fertigens des wenigstens einen abnehmbaren Teils (3) im Körper (2) aufweist.

11. Herstellungsverfahren gemäß Anspruch 10, **dadurch gekennzeichnet, daß** der Schritt (C) des Fertigens des wenigstens einen abnehmbaren Teils (3) einen Schritt des Schaffens der wenigstens einen Linie mit verringerter Festigkeit (32) im Körper (2) aufweist, um den wenigstens einen abnehmbaren Teil (3) abzugrenzen.

12. Herstellungsverfahren gemäß einem der Ansprüche 10 oder 11, **dadurch gekennzeichnet, daß** der Schritt (B) darin besteht,
- wenigstens eine unter den Geschmacksverbindungen oder den trigeminalen Verbindungen ausgewählte Verbindung von Interesse in Form einer Lösung, deren Lösungsmittel Wasser ist, oder
- wenigstens eine unter den Geruchsverbindungen ausgewählte Verbindung von Interesse in Form einer Lösung, deren Lösungsmittel Wasser oder eine wäßrig-alkoholische Lösung ist, abzulegen.

13. Herstellungsverfahren gemäß einem der Ansprüche 10 bis 12, **dadurch gekennzeichnet, daß** der Schritt (B) des Ablegens mittels einer Druckvorrichtung (8) vom Typ eines Tintenstrahldruckers ausgeführt wird, die Tanks (81), die geeignet sind, Flüssigkeiten zu enthalten, und Mittel (82) zur Emission der in den Tanks (81) enthaltenen Flüssigkeiten aufweist,
wobei die Tanks (81) jeweils
- wenigstens eine Verbindung von Interesse in einem Lösungsmittel gelöst,
- das Lösungsmittel allein und
- eine flüssige Tinte
enthalten.

## Claims

1. A device for evaluating the orosensory perception of a subject, which evaluation device (1) comprises a body (2) including at least one compound of interest liable to constitute an orosensory stimulus,
wherein the body (2) comprises at least one removable part (3) that, on the one hand, includes said at least one compound of interest, and on the other hand, is adapted to be separated from a residual part (21) of said body (2) to be deposited into the buccal cavity of said subject,
wherein the at least one removable part (3) is formed in said body (2), making a single-piece unit,
wherein the at least one removable part (3) is delimited by a peripheral edge (31) at least one portion of which is made integral with a residual part (21) of said body (2),
**characterized in that** said body (2) is made of an edible material, and
**in that** at least one portion of said peripheral edge (31) of said at least one removable part (3) is fastened with said residual part (21) of said body (2) by a line of least resistance (32).

2. The evaluation device according to claim 1, **characterized in that** said line of least resistance (32) consists in a pre-cut line.

3. The evaluation device according to any one of claims 1 or 2, **characterized in that** the body (2) is made from a material having a neutral or almost-neutral taste.

4. The evaluation device according to any one of claims 1 to 3, **characterized in that** the body (2) consists in a plate, preferably a sheet.

5. The evaluation device according to any one of claims 1 to 4, **characterized in that** the material constituting the body (2), including the removable parts (3), is unleavened paper.

6. The evaluation device according to any one of claims 1 to 5, **characterized in that** it includes at least one evaluation series (5) formed of at least two of said removable parts (3) :
- one at least of said removable parts (3) including the compound of interest or one at least of said compounds of interest, and
- the other one(s) of said removable parts (3) (i) being devoid of said compounds of interest or (ii) including said compound(s) of interest, in combination with at least one complementary compound liable to modify the orosensory perception.

7. The evaluation device according to any one of claims 1 to 6, **characterized in that** the body (2) includes a marking (7) that is formed so as to delimit and/or to indicate said at least one removable part (3) of said evaluation device (1).

8. The evaluation device according to any one of claims 1 to 7, **characterized in that** said at least one compound of interest is chosen among the compounds liable to generate a stimulus of gustatory and/or olfactory and/or trigeminal perception of the subject.

9. A system for evaluating the orosensory perception of a subject relative to at least one compound of interest liable to constitute an orosensory stimulus, wherein the evaluation unit (10) comprises:
(i) an evaluation device (1) according to any one of claims 1 to 8,
(ii) possibly a device (11) allowing a written collection of data relating to the orosensory perception of said subject during the implementation of said evaluation device (1), and
(iii) a computer program, recorded on a computer-readable support, comprising:
- computer-readable programming means for inputting said data relating to the orosensory perception of said subject and possibly for inputting personal data of said subject,
- computer-readable programming means for processing said data, adapted to determine an orosensory perception profile, and
- computer-readable programming means for providing said orosensory perception profile relating to said subject.
when said computer program is executed on a computer.

10. A method for manufacturing an evaluation device (1) according to any one of claims 1 to 9, **characterized in that** it comprises:
- a step (A) of providing the body (2) able to include said at least one compound of interest, and
- a step (B) of depositing said at least one compound of interest within at least one part of said body (2) that forms or that is intended to form said at least one removable part (3) of said body (2), wherein the method also includes a step (C) of making said at least one removable part (3) in the body (2).

11. The manufacturing method according to claim 10, **characterized in that** the step (C) of making said at least one removable part (3) comprises a step of making said at least one line of least resistance (32) in said body (2) to delimit said at least one removable part (3).

12. The manufacturing method according to any one of claims 10 or 11, **characterized in that** the deposit step (B) consists in depositing :
- at least one compound of interest chosen among the sapid compounds or the trigeminal compounds, as a solution whose solvent is consisted by water, or
- at least one compound of interest chosen among the odorous compounds, as a solution whose solvent is consisted by water or a water-alcohol solution.

13. The manufacturing method according to any one of claims 10 to 12, **characterized in that** the deposit step (B) is performed by means of a printing system (8) of the ink-jet type, comprising tanks (81) adapted to contain liquids and means (82) for spraying the liquids contained in said tanks (81),
wherein the tanks (81) respectively contain:
- at least one compound of interest in solution in a solvent,
- said solvent alone, and
- a liquid ink.
